# EUROPÄISCHE PATENTANMELDUNG

(11) **EP 0 588 192 A1**
(43) Veröffentlichungstag der Anmeldung: **23.03.1994**
(21) Anmeldenummer: 93114218.6
(22) Anmeldetag: 04.09.1993
(51) Int. Cl.: C07C 209/16, C07C 211/08, C07C 211/03

(54) **Verfahren zur Herstellung von tertiären Aminen**

(30) Priorität: 12.09.1992 DE 4230554
(71) Anmelder: BASF Aktiengesellschaft, D-67063 Ludwigshafen (DE)
(72) Erfinder: Mueller, Herbert, Dr., D-6710 Frankenthal (DE)

(57) **Zusammenfassung**

Verfahren zur Herstellung von Trialkylaminen der allgemeinen Formel I 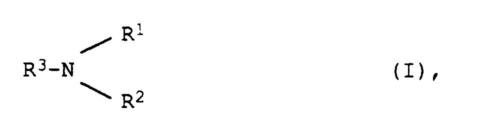
in der R¹, R² und R³ C₁- bis C₁₂-Alkyl bedeuten, durch Umsetzung von Dialkylaminen der allgemeinen Formel II 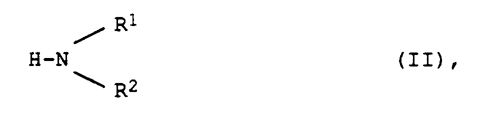
in der R¹ und R² C₁- bis C₁₂-Alkyl bedeuten, mit Alkoholen R³-OH in Gegenwart eines Kupferchromit-Erdalkalichromit enthaltenden Hydrierungs-/Dehydrierungskatalysators, indem man die Umsetzung in flüssiger Phase an Festbettkatalysatorem in Anwesenheit des bei der Reaktion gebildeten Wassers bei Temperaturen von 180 bis 210°C und Drücken von 40 bis 120 bar durchführt.

## Beschreibung

Die vorliegende Erfindung betrifft ein Verfahren zur Herstellung tertiärer Amine durch Umsetzung von sekundären Aminen mit Alkoholen in Gegenwart von Kupferchromit-Bariumchromit enthaltenden Hydrierungs-/Dehydrierungskatalysatoren bei erhöhten Temperaturen und Drücken.

Aus der DE-A-14 93 781 ist ein Verfahren zur Herstellung von tertiären Aminen bekannt, bei dem mindestens stöchiometrische Mengen eines sekundären Amins mit einem Alkohol umgesetzt werden. Ein Überschuß an Alkohol verschlechtert die Selektivität hinsichtlich der gebildeten tertiären Amine. Bei der Verwendung von primären Alkoholen werden geringe Umsätze und schlechte Selektivität erzielt.

Aus der US-A-3 708 539 ist ein Verfahren zur Herstellung von tertiären Aminen in flüssiger Phase bekannt, bei dem ein Alkohol mit einem sekundären Amin an Katalysatoren auf der Grundlage von Ruthenium, Osmium, Rhenium oder Technetium umgesetzt werden. Nachteil dieses Verfahrens besteht in den hohen Katalysatorrohstoffkosten.

Aus der DE-A-28 38 184 und der EP-A-24 225 sind Verfahren zur Herstellung tertiärer Amine durch Umsetzung von sekundären Aminen mit Alkoholen in Gegenwart von Kupferchromitkatalysatoren oder reinen Kupferkatalysatoren bekannt, die durch thermische Zersetzung und Reduktion von basischen Kupfer-Aluminium-Carbonaten herstellbar sind, durch Reaktion in der Gasphase z.B. aus Dimethylamin und den niedermolekularen Alkoholen.

Obwohl nach diesen beiden Verfahren die Synthese von z. B. Dimethylethylamin in technisch zufriedenstellender Weise auszuführen ist, bleibt die Synthese weiterhin verbesserungswürdig. Beiden Verfahren haftet der Nachteil an, daß für die Durchführung der Gasphasenreaktion ein verhältnismäßig hoher Energieaufwand für Verdampfen der Reaktionskomponenten aufgewendet werden muß, eine große Menge Wasserstoff und gasförmiges Dimethylamin im Kreis gefördert werden müssen und das Produkt beispielsweise Dimethylethylamin nur durch großen Kondensationsaufwand aus der geförderten Kreisgasmenge gewonnen werden kann. Meist ist eine hintereinander geschaltete mehrstufige Kondensation erforderlich. Da diese Verfahren in der Gasphase bei relativ niedrigem Wasserstoffpartialdruck an der nicht nur hydrierend sondern auch dehydrierend wirkenden Katalysatoren durchgeführt werden, bildet sich aus dem Alkohol auch der entsprechende Aldehyd, der durch Aldolkondensation zu unerwünschten Nebenprodukten führt, welche nicht nur die Ausbeute mindern, sondern auch als Verunreinigungen im Endprodukt erscheinen. Im Falle der Synthese des Ethyldimethylamins enthält der Rohaustrag z.B. Acetaldehyd. Das in der EP-A-24 225 beschriebene Verfahren sieht deshalb vor, daß das primär entstandene Reaktionsprodukt vor der endgültigen Aufarbeitung noch einer gesonderten Hydrierung zugeführt wird, in der der Acetaldehyd zum Ethanol hydriert wird, um Aufarbeitungsschwierigkeiten zu vermeiden.

Aus der EP-A-227 904 ist ein Verfahren zur Herstellung tertiärer Amine durch Umsetzung von sekundären Aminen mit Alkoholen in flüssiger Phase in Gegenwart von Alkalioxiden und/oder Erdalkalioxiden und/oder Alkalihydroxiden und/oder Erdalkalihydroxiden, in Anwesenheit von Wasser und Kupfer-Katalysatoren bekannt. Als Kupferkatalysatoren werden basische Kupfer-Aluminiumcarbonate empfohlen, die durch Fällung eines Kupfer-Aluminiumsalzes entstehen und anschließend thermisch zersetzt werden.

In der DE-A-25 35 073 wird ebenfalls die Flüssigphasen-Alkylierung von sekundären Aminen mit Alkoholen beschrieben. Als Katalysatoren werden Kupferchromit-Katalysatoren eingesetzt. Die Aminierung findet in der flüssigen Phase an suspendiertem Katalysator statt. Zum Gelingen der Reaktion ist es erforderlich, daß das Reaktionswasser im Zuge seiner Entstehung ständig aus dem Reaktionsgemisch entfernt wird.

Der vorliegenden Erfindung lag daher die Aufgabe zugrunde, den zuvor genannten Nachteilen abzuhelfen.

Demgemäß wurde ein neues und verbessertes Verfahren zur Herstellung von Trialkylaminen der allgemeinen Formel I 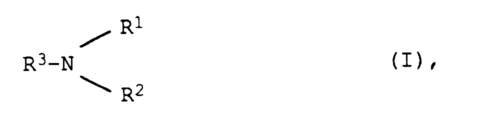
in der R¹, R² und R³ C₁- bis C₁₂-Alkyl bedeuten, durch Umsetzung von Dialkylaminen der allgemeinen Formel II 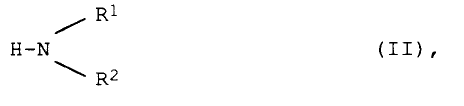
in der R¹ und R² C₁- bis C₁₂-Alkyl bedeuten, mit Alkoholen R³-OH in Gegenwart eines Kupferchromit-Erdalkalichromit enthaltenden Hydrierungs-/Dehydrierungskatalysators gefunden, welches dadurch gekennzeichnet ist, daß man die Umsetzung in flüssiger Phase an Festbettkatalysatoren in Anwesenheit des bei der Reaktion gebildeten Wassers bei Temperaturen von 180 bis 210°C und Drücken von 40 bis 120 bar durchführt.

Das erfindungsgemäße Verfahren kann wie folgt durchgeführt werden:
Die sekundären Amine II können mit einem Alkohol R³-OH bei Temperaturen von 160 bis 250°C, bevorzugt 170 bis 210°C im allgemeinen unter Überdruck wie z.B. unter einem Gesamtdruck von 40 bis 120 bar, bevorzugt 50 bis 100 bar, besonders bevorzugt 60 bis 100 bar und einem Wasserstoffpartialdruck von 10 bis 50 bar, bevorzugt 15 bis 40 bar, besonders bevorzugt 20 bis 35 bar umgesetzt werden. Der Gesamtdruck der Reaktion setzt sich aus dem Dampfdruck der Reaktionspartner und dem Wasserstoffpartialdruck zusammen. Auf den Auslaß von Abgas kann in der Regel weitgehend verzichtet werden, da sich nach dem erfindungsgemäßen Verfahren fremde Inertgase praktisch nicht bilden. Am Ofenende kann das Reaktionsprodukt in einer Vorlage, die standgeregelt betrieben werden kann, kontinuierlich entnommen werden. Die Reaktion ist mit ca. 7 Kcal/mol leicht exotherm. Deshalb ist es leicht das Reaktionsgefäß isotherm zu halten, indem man einen Teil des Umsetzungsproduktes mittels einer Kreispumpe nach vorhergehender Kühlung auf die Katalysatorschüttung zurückführt. Der Reaktionsaustrag kann noch nicht umgesetztes sekundäres Amin II und nicht umgesetzten Alkohol enthalten. Beide lassen sich destillativ abtrennen und erneut in die erfindungsgemäße Reaktion einsetzten.

Das Molverhältnis von sekundärem Amin II zum Alkohol beträgt in der Regel 0,05 : 1 bis 50 : 1, bevorzugt 0,5 : 1 bis 10 : 1 besonders bevorzugt 1 : 1 bis 3 : 1.

Das Verfahren kann an Festbettkatalysatoren bevorzugt in Sumpffahrweise durchgeführt werden.

An Festbettkatalysatoren arbeitet man zweckmäßig folgendermaßen:
Als Reaktor verwendet man z.B. ein senkrecht angeordnetes zylindrisches Gefäß mit den üblichen Einrichtungen zur Kühlung und Heizung und der Zufuhrmöglichkeit der Reaktionsteilnehmer. Das Reaktionsgefäß kann mit dem Katalysator, der beliebig geformt sein kann, gefüllt werden. Häufig verwendet man den Katalysator als Strangpreßling mit einem Durchmesser von 3 bis 6 mm und einer Länge bis zu 5 cm. Er kann aber auch tablettiert in Form von Zylindern mit den Maßen von z.B. 5 mm Durchmesser und 5 mm Höhe oder als Kugeln oder in jeder anderen beliebigen Form eingesetzt werden.

In der bevorzugten Ausführungsform des erfindungsgemäßen Verfahrens verwendet man Kupferchromit / Bariumchromit-Katalysatoren deren Bariumgehalt > 1 Gew.-% inbesondere > 3 Gew.-% sein soll.

Ein für die Aminierung sehr gut geeigneter Katalysator ist der G 22, der Süd-Chemie, der neben Sauerstoff 33 Gew.-% Kupfer, 27 Gew.-% Chrom und 11 Gew.-% Barium enthält. Wird in den Katalysatoren Barium durch beispielsweise Magnesium oder Kalzium ersetzt, so weisen diese nur ca. 60 % der Aktivität und ca. 90 % der Selektivität der bariumhaltigen Katalysatoren auf. Die Herstellung der Katalysatoren ist aus dem Handbuch "Houben-Weyl, Methoden der organischen Chemie, 4. Auflage, 1955, Georg Thieme Verlag Stuttgart, in Band 4/2, Seite 180 bis 183 bekannt. Statt Bariumchromit können die Katalysatoren auch Calzium- oder Magnesiumchromit enthalten.

Der oben genannte Katalysator G 22 kann in Tablettenform, 3 x 3 und 4,5 x 4,5 mm, verwendet werden. Der Katalysator wird zweckmäßiger Weise vor der Verwendung durch Reduktion mit Wasserstoff bei Temperaturen zwischen 160 und 220°C aktiviert.

Obwohl die Verwendung des Katalysators mit hohem Bariumgehalt bereits eine ausreichende Selektivität liefert, ist es eine wünschenswerte Maßnahme beim vorliegenden Verfahren, den Reaktionspartnern ein Alkalioxid, ein Erdalkalioxid, ein Alkalihydroxid und/oder ein Erdalkalihydroxid zuzugeben. Durch diese Maßnahme gelingt es, Umalkylierungsreaktionen und Disproportionierungen besonders vorteilhaft zu unterdrücken. Dabei war es überraschend, daß diese Wirkung verstärkt eintritt, wenn die Alkali- bzw. Erdalkaliverbindung den flüssigen Reaktionspartnern in kontinuierlicher Weise zugeführt wird. Diese Wirkung ist weniger stark ausgeprägt, wenn der Alkaligehalt dem Katalysator-Formkörper zugeführt wird.

Die Reaktionsgeschwindigkeit ist sehr hoch, es lassen sich ohne weiteres 100 bis 300 Gew.-Teile tertiäres Amin I pro Liter Katalysatorvolumen in der Stunde erzeugen.

Die Katalysatoren behalten in der Regel beim erfindungsgemäßen Verfahren über lange Zeiträume (z.B. 3 bis 5 Jahre) ihre Aktivität und Selektivität.

Die Substituenten R¹, R² und R³ in den Verbindungen I und II sowie im Alkohol R³OH haben folgende Bedeutungen:
R¹, R² und R³
- unabhängig voneinander
- C₁- bis C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl,iso-Decyl, n-Undecyl, iso-Undecyl, n-Dodecyl und iso-Dodecyl,bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl und iso-Octyl, besonders bevorzugt C₁- bis C₄-Alkyl wie Methyl, Ethyl, n-Propyl, iso-Propyl, n-Butyl, iso-Butyl, sec.-Butyl und tert.-Butyl.

Als Alkohole R³-OH eignen sich insbesondere einwertige aliphatische Alkohole R³-OH z. B. Ethanol, n-Propanol, i-Propanol, n-Butanol oder sec.-Butanol, Hexanol und Decanol.

Als sekundäre Amine II eignen sich z. B. Dimethylamin, Methylethylamin, Diethylamin, Diisopropylamin, Di-n-butylamin und Ethyl-n-hexylamin.

Tertiäre Amine I vom Typ Dimethylfettalkylamin, deren Fettalkyl-Rest im allgemeinen eine C-Zahl > 8 aufweist, besitzen besonderes technisches Interesse für die Herstellung quartärer Ammoniumverbindungen, z . B. Phasentransferkatalysatoren-Weichspüler oder Bakterizide (Römps Chemielexikon, 8. Auflage, 1987, Bd. 5, Seite 3438-3439) .

Niedermolekulare tertiäre Trialkylamine I werden industriell in großem Umfang benützt, z.B. als Katalysatoren für die Herstellung von Polyurethanen oder Epoxiharzen.

### Beispiele

Die genannten Teile sind Gew.-Teile, sie verhalten sich zu Vol.-Teilen wie kg zu l.

### Beispiel 1

Für die kontinuierliche Herstellung des Ethyldimethylamins wurde verflüssigtes Dimethylamin technischer Qualität und mit 1,2 % Toluol vergälltes Ethanolazeotrop (95,6 % Ethanol, 4,4 % Wasser) verwendet.

Die Aminierung wurde in einem senkrecht stehenden Reaktionsrohr mit 1000 Vol-Teilen Reaktionsinhalt durchgeführt. Das Verhältnis des Durchmessers zur Länge des Reaktionsrohres betrug 1:40. Mittels eines organischen Wärmeträgers, der in einem Heizmantel umgepumpt wurde, ließ sich das Reaktionsrohr thermostatisieren. In das Reaktionsrohr wurden 700 Vol-Teile des Katalysators G 22 (Süd-Chemie AG) gefüllt. Der Katalysator hatte die Form von Zylindern, die 3 mm Höhe und 3 mm Durchmesser maßen. Der Katalysator war reduziert, also aktiviert und vor Aktivitätsverlust durch Überstellung mit Dekanol geschützt.

Wird der Katalysator in nicht voraktivierter Form verwendet, so kann er auf folgende Weise aktiviert und mit Wasserstoff reduziert werden: Man rieselt stündlich etwa 300 Vol-Teile Ethanol über den Katalysator und führt diesen von unten nach oben bei 180 bis 200°C zunächst ein Stickstoff-/Wasserstoffgemisch und später reinen Wasserstoff zu. Die Aktivierung des Katalysators ist beendet, wenn im Ethanolazeotrop kein Reduktionswasser zusätzlich nachweisbar ist.

Für die Durchführung der Aminierung werden dem Ofen dann bei 200°C stündlich 300 Teile Ethanol und 100 Teile Dimethylamin bei 50 bar Gesamtdruck (Wasserstoffpartialdruck ca. 10 bar) von unten nach oben zugefahren. Am Kopf der Vorlage wurden stündlich ca. 0,1 Normalvolumenteile Wasserstoff als Abgas gefahren. Das die Vorlage verlassende Reaktionsprodukt hatte nach gaschromatographischer Analyse und Destillationsanalyse wasserfrei gerechnet folgende Zusammensetzung:

| | |
|---|---|
| Trimethylamin: | < 0,1 Gew.-% |
| Methylethylamin: | < 0,1 Gew.-% |
| Dimethylamin: | 0,2 Gew.-% |
| Dimethylethylamin: | 60 Gew.-% |
| Diethylmethylamin: | < 0,1 Gew.-% |
| Ethanol: | 38 Gew.-% |
| Toluol: | 1,5 Gew.-% |

Durch Destillation läßt sich aus diesem Rohgemisch Dimethylethylamin mit über 99,8 % Reinheit gewinnen.

### Beispiel 2

Man arbeitet wie in Beispiel 1 beschrieben und alkyliert ein Gemisch das aus 8 Teilen Diethylamin und 1 Teil Ethanol besteht. Der Gesamtdruck (Partialdruck des Reaktionsgemisches und Wasserstoffpartialdruck) beträgt 50 bar, die Reaktionstemperatur 210°C. Auf 700 Vol-Teile des Katalysators werden von unten nach oben stündlich 1000 Vol-Teile des Gemisches aus Diethylamin und Ethanol gegeben. Es entsteht ein Reaktionsprodukt, das wasserfrei gerechnet folgende gaschromatographische Zusammensetzung besitzt:

| | |
|---|---|
| Unbekannte: | < 0,1 Fl-% |
| Monoethylamin: | < 0,1 Fl-% |
| Diethylamin: | 48,9 Fl-% |
| Triethylamin: | 49,6 Fl-% |
| Ethanol: | 0,2 Fl-% |

## Patentansprüche

1. Verfahren zur Herstellung von Trialkylaminen der allgemeinen Formel I 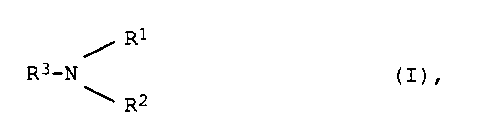 in der R¹, R² und R³ C₁- bis C₁₂-Alkyl bedeuten, durch Umsetzung von Dialkylaminen der allgemeinen Formel II 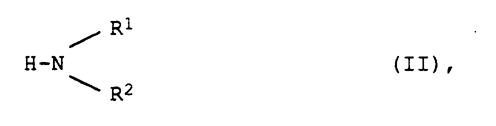 in der R¹ und R² C₁- bis C₁₂-Alkyl bedeuten, mit Alkoholen R³-OH in Gegenwart eines Kupferchromit-Erdalkalichromit enthaltenden Hydrierungs-/Dehydrierungskatalysators, dadurch gekennzeichnet, daß man die Umsetzung in flüssiger Phase an Festbettkatalysatoren in Anwesenheit des bei der Reaktion gebildeten Wassers bei Temperaturen von 180 bis 210°C und Drücken von 40 bis 120 bar durchführt.

2. Verfahren zur Herstellung von Trialkylaminen I nach Anspruch 1, dadurch gekennzeichnet, daß man die Umsetzung in Gegenwart von Wasserstoff durchführt.

3. Verfahren zur Herstellung von Trialkylaminen I nach Anspruch 1, dadurch gekennzeichnet, daß man die Umsetzung bei einem Wasserstoffpartialdruck von 10 bis 50 bar durchführt.

4. Verfahren zur Herstellung von Trialkylaminen I nach Anspruch 1, dadurch gekennzeichnet, daß man die Umsetzung in Gegenwart von 0,001 bis 2 Gew.-% eines Alkalioxids, eines Erdalkalioxids, eines Alkalihydroxids und/oder Erdalkalihydroxids durchführt.
